# EUROPEAN PATENT APPLICATION

(11) **EP 1 270 596 A1**
(43) Date of publication of application: **02.01.2003**
(21) Application number: 01917858.1
(22) Date of filing: 06.04.2001
(51) Int. Cl.: C07K 17/06, G01N 33/543, G01N 33/544, G01N 33/545, G01N 33/546, G01N 33/547, G01N 33/548, G01N 33/549, G01N 33/551, G01N 33/552, G01N 33/553, G01N 33/554, G01N 33/555, G01N 33/556

(54) **ANTIBODY/CARRIER COMPLEX, PROCESS FOR PRODUCING THE SAME, METHOD OF CONTROLLING ANTIGEN-ANTIBODY REACTION BY USING THE SAME AND IMMUNOASSAY METHOD**

(30) Priority: 07.04.2000 JP 2000106019
(71) Applicant: MATSUSHITA ELECTRIC INDUSTRIAL CO., LTD., Kadoma-shi, Osaka 571-8501 (JP)
(72) Inventor: HIRAI, Mahito, Kyotanabe-shi, Kyoto 610-0341 (JP); SHIGETOH, Nobuyuki, Kyotanabe-shi, Kyoto 610-0354 (JP)
(74) Representative: Gallagher, Kirk James
(86) International application number: JP0103017
(87) International publication number: WO01077182

(57) **Abstract**

An antibody/carrier complex which makes it possible to easily control the reactivity in an antigen-antibody reaction without producing a new antibody. This antibody/carrier complex is a complex containing at least one antibody and has a polymer structure wherein each antigen-binding site of the antibody is allowed to be arranged so as to react with the antigen.

## Description

### TECHNICAL FIELD

The present invention relates to an antibody/carrier complex used for immunological analysis, measurement, assays, or the like, using an antigen-antibody reaction, such as immunochromatography, affinity chromatography, blotting, immunoturbidimetry (immunonephelometry), dyeing of an antigen in a tissue using an antibody, Ouchterlony, enzyme immunoassay, etc., in the field of medicine, and the like.

### BACKGROUND ART

In immunological analysis, measurement, assays, or the like, which uses an antigen-antibody reaction, the measurable concentration range of an antigen depends on the affinity of the antibody. Thus, in order to obtain an antibody having an affinity which corresponds to the concentration range of an antigen as a subject of analysis, measurement, or assay, it was conventionally necessary to search for and obtain an antibody having the intended affinity among commercially-available antibodies, or necessary to produce a novel antibody having the intended affinity.

### DISCLOSURE OF THE INVENTION

However, an antibody having an affinity corresponding to the concentration range of an antigen as a subject of analysis cannot be obtained in some cases even when the production method is improved, because the adjustable range of the affinity of an obtained monoclonal or polyclonal antibody itself is finite.

In view of the above problem, the present invention is directed to an objective of providing: an antibody/carrier complex wherein the affinity of an antibody to an antigen, i.e., the reactivity of an antigen-antibody reaction, can readily be controlled without producing a novel antibody; a production method of the same; a method for controlling an antigen-antibody reaction using the same; and an immunological measurement method.

In order to solve the above problem, an antibody/carrier complex of the present invention includes at least one antibody, the antibody/carrier complex including a polymer structure wherein each antigen binding site of the antibody is allowed to be arranged so as to react with an antigen.

Preferably, the polymer structure is an antibody polymer or carrier polymer.

Preferably, the carrier is selected from a group consisting of protein, carbohydrate, and lipid.

Preferably, the carrier is bonded to a Fc-site of the antibody.

The present invention relates also to a method for producing the above antibody/carrier complex. This method comprises a step of copolymerizing an antibody and a carrier using a polymerization reagent.

The present invention relates also to a method for producing the above antibody/carrier complex. This method comprises: a step of polymerizing a carrier using a polymerization reagent so as to form a carrier polymer; and a step of bonding an antibody to the carrier polymer.

The present invention relates also to a method for producing the above antibody/carrier complex. This method comprises: a step of polymerizing an antibody using a polymerization reagent so as to form an antibody polymer; and a step of bonding a carrier to the antibody polymer.

The present invention relates also to a method for producing the above antibody/carrier complex. This method comprises: a step of polymerizing a carrier using a polymerization reagent so as to form a carrier polymer; a step of polymerizing an antibody using a polymerization reagent so as to form an antibody polymer; and a step of bonding the antibody polymer and the carrier polymer.

Preferably, the polymerization reagent is 3,3'-dithiobis(sulfosuccinimidylpropionate) or dithiothreitol.

One aspect of the present invention relates to a method for adjusting the reactivity of an antigen-antibody reaction. This method comprises steps of: in a reaction of an antibody and a polymerization reagent, changing a parameter selected from a group consisting of the equivalent of the polymerization reagent with respect to the antibody, reaction temperature, reaction time, and the concentration of antibody, so as to obtain a plurality of antibody polymers having different degrees of polymerization; bonding the plurality of antibody polymers to carriers so as to obtain a set of antibody/carrier complexes; and selecting an antibody/carrier complex which reacts with an antigen at a desired degree of reaction, from the set of antibody/carrier complexes.

One aspect of the present invention relates to an immunological measurement method. This method comprises a step of bringing a subject substance in a sample and the above antibody/carrier complex into an antigen-antibody reaction.

### BRIEF DESCRIPTION OF THE DRAWINGS

Figure **1** is a diagram showing a chromatogram for an anti-hCG antibody before polymerization used in an embodiment of the present invention, which was measured by high-performance liquid chromatography.
Figure **2** is a diagram showing a chromatogram for one antibody polymer according to an embodiment of the present invention, which was measured by high-performance liquid chromatography.
Figure **3** is a diagram showing a chromatogram for another antibody polymer according to an embodiment of the present invention, which was measured by high-performance liquid chromatography.
Figure **4** is a diagram showing a chromatogram for another antibody polymer according to an embodiment of the present invention, which was measured by high-performance liquid chromatography.
Figure **5** is a diagram showing a chromatogram for another antibody polymer according to an embodiment of the present invention, which was measured by high-performance liquid chromatography.
Figure **6** is a diagram showing a chromatogram for a carrier polymer according to an embodiment of the present invention, which was measured by high-performance liquid chromatography.
Figure **7** is a diagram showing a chromatogram for an antibody/carrier polymer complex according to an embodiment of the present invention, which was measured by high-performance liquid chromatography.
Figure **8** is a perspective view showing an immunochromatography device used in an immunological measurement method according to an embodiment of the present invention.

### BEST MODE FOR CARRYING OUT THE INVENTION

Hereinafter, the present invention will be described in detail.

It should be noted that in the present invention, protein separation and analyzing methods and immunological methods known in the subject art fields are adopted unless otherwise noted. These methods can be performed using commercially-available enzymes, kits, antibodies, labeling substances, etc.

As described above, an antibody/carrier complex of the present invention includes at least one antibody, and is characterized in that the antibody/carrier complex comprises a polymer structure which allows each antigen binding site of the antibody to be placed so as to react with an antigen. Such an antibody/carrier complex has many antigen binding sites in comparison to a bivalent antibody which does not have a polymer structure. In the antibody/carrier complex of the present invention, the ratio of antigen binding sites which can effectively participate in antigen-antibody reactions to the total antigen binding sites can be changed. Thus, the antibody/carrier complex of the present invention can be used to adjust the reactivity of an antigen-antibody reaction so as to be higher or lower in comparison to an immunoreaction of unpolymerized bivalent antibody and antigen. Further, the antibody/carrier complex of the present invention has a larger size in comparison to an antibody polymer formed of the same amount of antibody without a carrier. Thus, the number of antigen binding sites, which are present on the surface of a complex molecule and at which a reaction with an antigen is not sterically inhibited, can be increased, and as a result, the reactivity range of an antigen-antibody reaction can be extended towards higher reactivity. Further, when an antibody/carrier complex of the present invention has the same size as that of an antibody polymer formed of only an antibody, the amount of the antibody is smaller than that of the antibody polymer, while the antibody/carrier complex achieves an antigen-antibody reaction in substantially the same reactivity range as that achieved by the antibody polymer. One of the antibody/carrier complexes which is most efficiently modified so as to have high reactivity has a structure where the core portion thereof is formed of a carrier or a carrier polymer, and one or more antibody molecules are bonded onto the surface of the core portion such that antibody binding sites are exposed outward. Furthermore, in the case where an antibody/carrier complex of the present invention is labeled with a labeling substance for use in an immunological analysis or the like, only a carrier portion of the antibody/carrier complex can be labeled, and therefore, there is little possibility that an antigen-antibody reaction is inhibited by a labeled antibody. As a result, the number of antigen binding sites, which are present on the surface of a complex molecule and at which a reaction with an antigen is not sterically inhibited, can be increased, and as a result, the reactivity range of an antigen-antibody reaction of the antibody/carrier complex can be extended towards higher reactivity in comparison to when an antibody polymer formed of only an antibody is used.

The present invention is not limited to a specific antibody. Any antibody can be used regardless of its origin, subclass, etc. For example, IgG, IgM, IgA, IgE, and IgD derived from a human or mouse, and IgG, IgM, IgA, IgE, IgD, and IgY derived from a rat, rabbit, goat, sheep, chicken, or the like, may be used. These antibodies may be bought as commercially-available products, or may be extracted directly from an animal.

The structure of a carrier of the antibody/carrier complex may be formed of any material so long as the material does not inhibit an antigen-antibody reaction. Preferably. it may be a material which is found in a body of an organism. Protein, carbohydrate, and lipid may be used. Examples of such a material include proteins which do not function as an antibody, such as serum protein, gelatin, or the like, carbohydrate such as monosaccharides, oligosaccharides, polysaccharides, complex carbohydrates, or the like, and lipids such as simple lipid. complex lipid, lipid derivative, or the like. Among these, protein and carbohydrate are preferable because a polymerization reaction can readily be caused. Further, proteins which do not function as an enzyme are preferable because such protein does not cause a reaction with a substance other than a subject antigen. Furthermore, albumin derived from serum, or the like, is also preferable because such albumin does not inhibit a reaction of an antibody and has high water-solubility.

Furthermore, it is preferable that a carrier is capable of bonding to a Fc-site of an antibody. In such a case, the antibody and the carrier are combined such that an antigen binding site faces in a direction opposite to the carrier. Thus, the possibility that an antigen-antibody reaction is inhibited by the bonding of the antibody and carrier is reduced, and therefore, the adjustable reactivity range of the antibody can be extended towards higher reactivity. The carrier bonding to the Fc-site of the antibody is, for example, protein A, protein G, or the like.

A production method of an antibody/carrier complex according to the present invention is characterized by copolymerizing an antibody and a carrier using a polymerization reagent.

The production method may include a step of polymerizing a carrier using a polymerization reagent so as to form a carrier polymer, and a step of bonding an antibody to the carrier polymer.

Alternatively, the production method may include a step of polymerizing an antibody using a polymerization reagent so as to form an antibody polymer, and a step of bonding an carrier to the antibody polymer.

Alternatively, the production method may include a step of polymerizing a carrier using a polymerization reagent so as to form a carrier polymer, a step of polymerizing an antibody using a polymerization reagent so as to form an antibody polymer, and a step of bonding the carrier polymer and the antibody polymer.

Among these variations, when the production method of an antibody/carrier complex, which includes a step of polymerizing a carrier using a polymerization reagent so as to form a carrier polymer and a step of bonding an antibody to the carrier polymer, is used, an antibody/carrier complex which has the carrier polymer in a core portion and the antibody in a surface portion can be readily formed. This method is preferable because the adjustable affinity range of the antibody can be extended towards higher affinity.

The polymerization reagent is not limited to any specific reagent so long as the reagent can cause a polymerization reaction. For example, as a polymerization reagent for an antibody, a polyfunctional reagent having two or more functional groups which are capable of bonding to an antibody (succinimidyl group, pyridyldisulfide group, or the like) in one molecule, a reagent which reduces a disulfide bond of protein, etc., may be used.

The polyfunctional reagent may be, for example, 3,3'-dithiobis(sulfosuccinimidylpropionate)(hereinafter, referred to as "DTSSP"), bis(sulfosuccinimidyl)suberate, disulfosuccinimidyltartrate, ethylene glycol bis(sulfosuccinimidylsuccinate), N-succinimidyl-3-(2-pyridyldithio)propionate, bis[2-(sulfosuccinimidoxycarbonyloxy)ethyl]sulfone, dimethylsuberimidate ^{·} 2HCl, N-γ-maleimidobutyryloxysulfosuccimide ester, sulfosuccimidi[4-iodoacetyl], bismaleimidohexane, 1,4-di-[3'-(2'-pyridyldithio)propione amide]butane, 2-mercaptomethylamine ^{·} HCl, dithiothreitol (hereinafter, referred to as "DTT"), etc.

Among the above, it is preferable to use DTSSP or DTT as a polyfunctional reagent in the respect that a reaction with an antibody can readily be controlled.

A reagent which reduces disulfide bonds of proteins may be, for example, 2-mercaptoethanol, or the like.

A method for adjusting the reactivity of an antigen-antibody reaction is characterized by controlling the degree of polymerization of the antibody in the above antibody/carrier complex.

In the antibody/carrier complex of the present invention, the affinity to an antigen in an antigen-antibody reaction increases as the degree of polymerization of an antibody increases, but if the degree of polymerization is increased after the highest affinity has been reached, the affinity decreases to a level lower than the affinity of an antibody achieved before polymerization. This is because the number of antigen binding sites of the antibody/carrier complex increases along with an increase in the degree of polymerization of the antigen, and accordingly, the reactivity of the reaction with the antigen increases; whereas a further increase in the degree of polymerization accelerates polymerization via antigen binding sites, or inhibits sterically binding of antigen binding sites to antigens, and accordingly, the reactivity of the reaction with the antigen is decreased. Thus, by controlling the degree of polymerization of an antibody in an antibody/carrier complex. the affinity of the antibody to the antigen can be adjusted.

Furthermore, an immunological measurement method of the present invention is characterized by measurement of a subject substance, using an antigen-antibody reaction of the subject substance in a sample with the above antibody/carrier complex. As the immunological measurement method, a conventionally known method can be used without any special limitation. The immunological measurement method may be, for example, immunochromatography, affinity chromatography, blotting, immunoturbidimetry (immunonephelometry), dyeing of an antigen in a tissue using an antibody, Ouchterlony, enzyme immunoassay, etc.

### (Examples)

Hereinafter, the present invention is specifically described with reference to Examples, but is not limited to the following Examples.

### (Example 1)

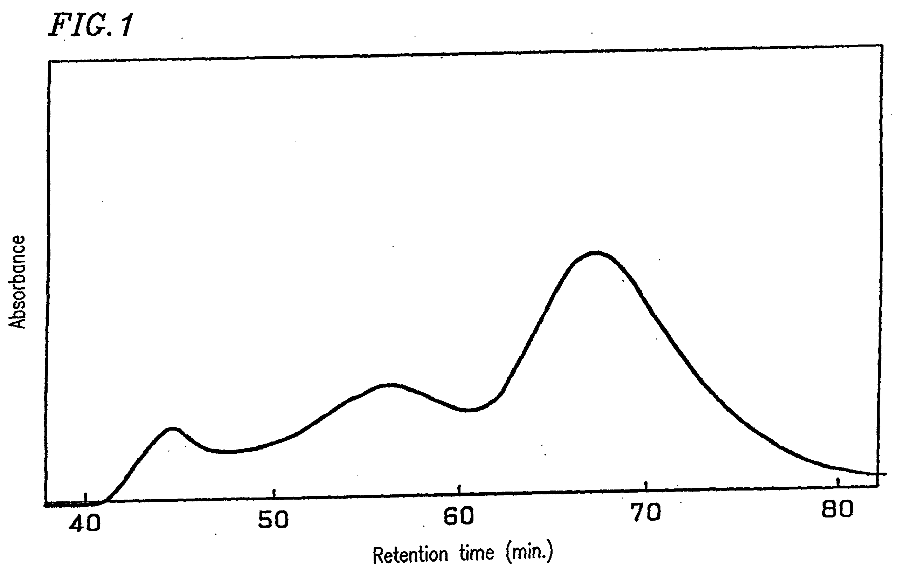

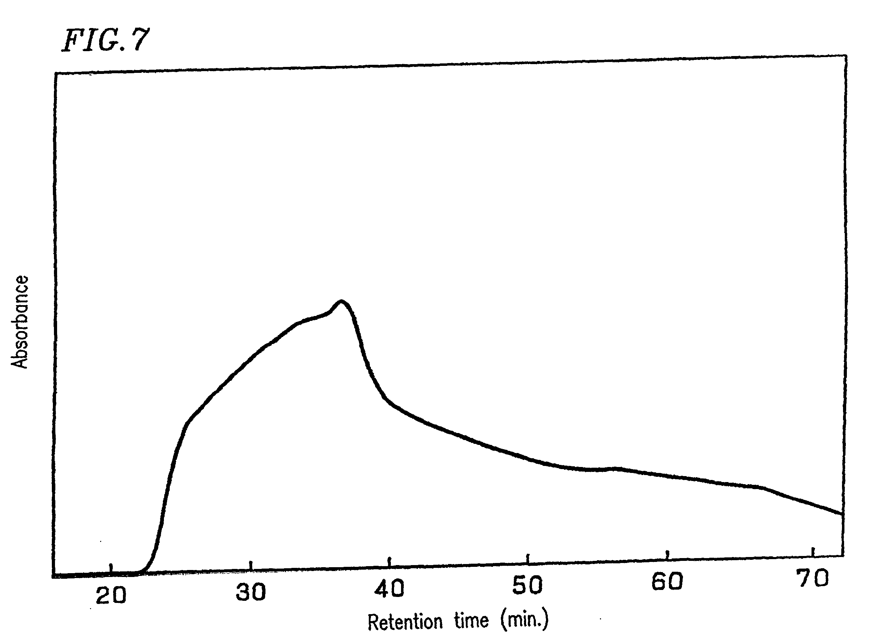

Hereinafter, an example of the present invention is described with reference to Figures **1** and **7**.

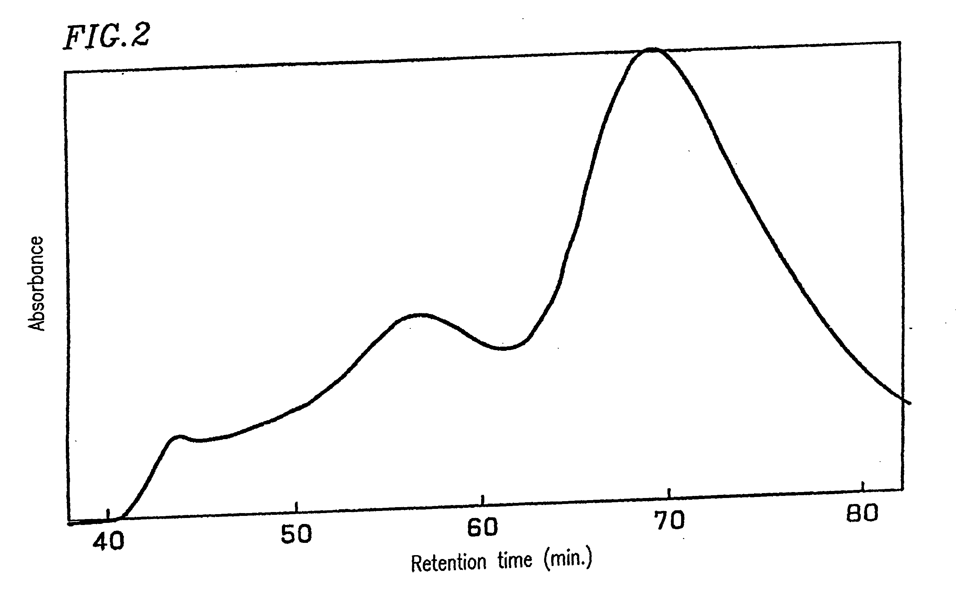

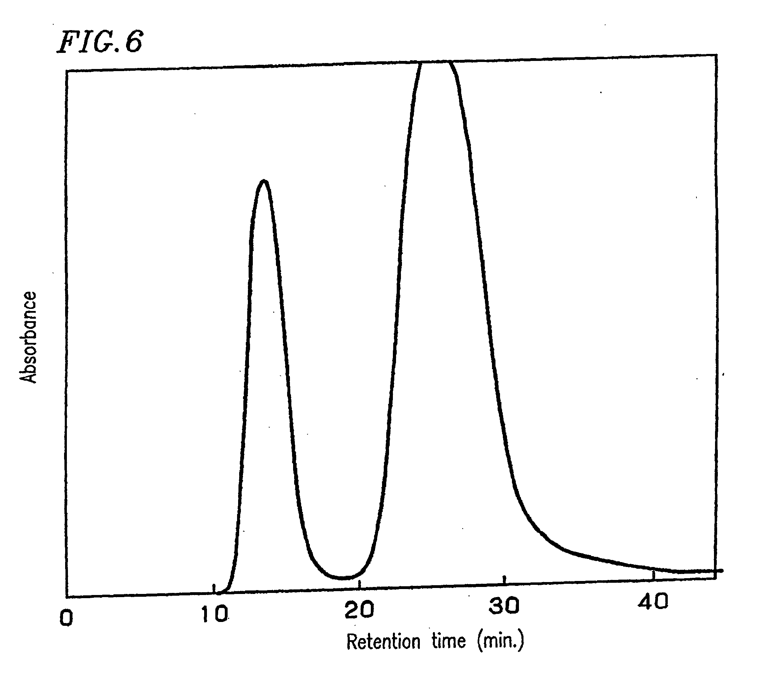

Figure **1** is a chromatogram measured for an unpolymerized antibody by high-performance liquid chromatography. Figures **2** through **5** are chromatograms measured for an antibody complex of this Example by high-performance liquid chromatography. Figure **6** is a chromatogram measured for a carrier polymer of this Example by high-performance liquid chromatography. Figure **7** is a chromatogram measured for an antibody/carrier complex of this Example by high-performance liquid chromatography.

### 1. Production of antibody polymer

First, an antibody polymer was produced. An antibody directed to a human chorionic gonadotrophin (hereinafter, "hGC"), which is hereinafter referred to as an anti-hGC antibody, was used as the antibody. 10 mg of anti-hGC antibody was added into 1 ml phosphate buffered saline (hereinafter, "PBS"), to a concentration of 10 mg/ml. To this prepared solution, DTSSP having different equivalents, equivalents of 20, 50, 100, and 200, were added as polyfunctional polymerization reagents, and the reactions in the reagents were then performed for 30 minutes so as to polymerize the antibody so as to obtain Solution (A), Solution (B), Solution (C), and Solution (D). Sephacryl S-300HR Column (produced by Amersham Pharmacia) was used in high-performance liquid chromatography (hereinafter, HPLC) to evaluate the degree of polymerization of the antibody, and to purify the antibody under the condition of flow velocity of 150 ml/h. Figures **1** through **5** show resultant chromatograms of the HPLC. The vertical axis represents the absorbance (relative value), and the horizontal axis represents the retention time. As a molecular weight becomes greater, the retention time becomes shorter, and as a result, the peak of the chromatogram shifts leftward.

Figure **1** shows a chromatogram of an unpolymerized, anti-hCG antibody. The peak of the anti-hCG antibody occurs near the retention time range of 66-68 minutes. Small peaks are found near the times of 44 minutes and 56 minutes, because the antibodies were denatured and aggregated in the solution so that the molecular weight appeared greater.

Figure **2** shows a chromatogram for Solution (A) where the antibody was brought into reaction using the DTSSP having an equivalent of 20 to the antibody. Polymerization had not almost advanced yet , so that no change was found in the pattern of the chromatogram, and the peak occurred near the retention time of 70 minutes, as seen in Figure **1**.

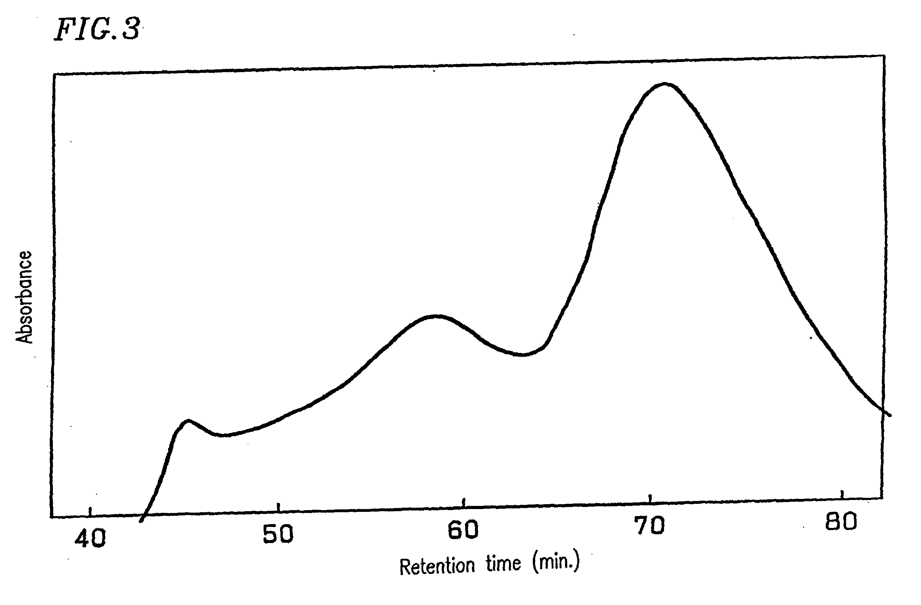

Figure **3** shows a chromatogram for Solution (B) where the antibody was brought into reaction using the DTSSP having an equivalent of 50 to the antibody. Polymerization of the antibody had slightly advanced, so that the peak near the retention time of 45 minutes was slightly increased.

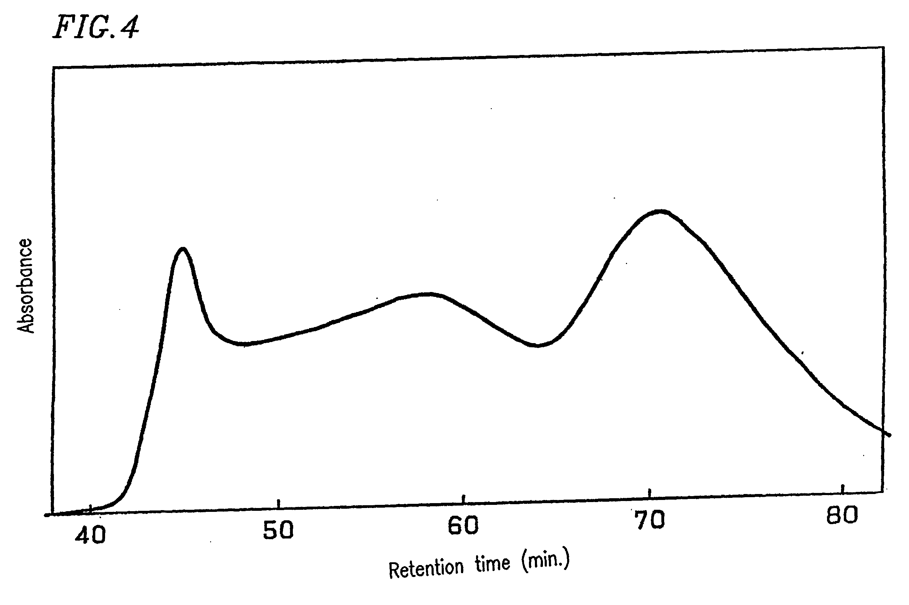

Figure **4** shows a chromatogram for Solution (C) where the antibody was brought into reaction using the DTSSP having an equivalent of 100 to the antibody. Polymerization of the antibody had further advanced, so that a large peak occurred near the retention time of 45 minutes.

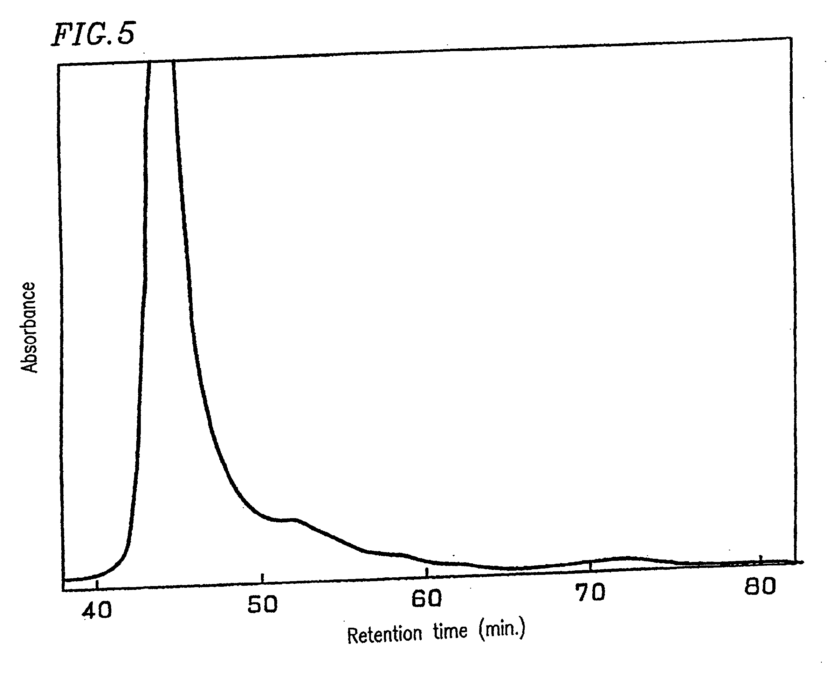

Figure **5** shows a chromatogram for Solution (D) where the antibody was brought into reaction using the DTSSP having an equivalent of 200 to the antibody. Polymerization of the antibody had further advanced, so that the peak near the retention time of 70 minutes disappeared, and a very large peak occurred near the retention time of 45 minutes.

The molecular weight of the antibody complex corresponding to the peak near the retention time of 45 minutes is considered to be equal to or greater than the exclusion limit of the column, i.e., equal to or greater than the molecular weight of 2×10⁶. Considering that the peak near the retention time of 45 minutes was increased by increasing the equivalent of the polyfunctional reagent, it is understood that the proportion of the antibodies having greater degree of polymerization was increased.

Based on the above results, the degree of polymerization of the antibodies of the obtained antibody polymer could be adjusted by maintaining the reaction time constant and controlling the equivalent of the polyfunctional reagent added.

Next, the antibody polymer was produced for use in production of an antibody/carrier complex. First, a PBS solution having an anti-hCG antibody concentration of 10 mg/ml was prepared. 4.056 mg of DTSSP, which has an equivalent of 100 with respect to 10 mg of antibody, was dissolved in 100 µl of PBS. 1 ml of antibody solution and 100 µl of DTSSP solution are slowly stirred at 35°C for 30 minutes. After the reaction has been completed, a Sephadex G-25 Column (produced by Amersham Pharmacia) was used to extract from the reaction solution a fraction including an antibody polymer.

### 2. Production of carrier polymer

Bovine serum albumin (hereinafter, referred to as BSA) was used as a carrier. 110 mg of BSA was dissolved in 5 ml PBS so as to prepare a BSA solution. This amount of BSA corresponds to equivalent of 25 with respect to 10 mg of antibody. Then, a PBS solution having a DTT concentration of 77 mg/ml was prepared. The 1 ml of PBS solution having such a DTT concentration was mixed with 5 ml of PBS solution containing BSA, the mixture was slowly stirred at room temperature for 30 to 40 minutes, and the reaction was stopped immediately before the solution became cloudy. After the reaction was completed, a G-25 Column was used to collect from the reaction solution a fraction including a carrier polymer (retention time of 12 to 18 minutes) as shown in Figure **6**.

### 3. Production of antibody/carrier complex

A carrier polymer is fractionated, and immediately thereafter, mixed and brought into reaction with the previously-fractionated antibody polymer. The reaction was performed overnight at 4°C. This reaction advanced only by mixing the carrier polymer and the antibody polymer. This was supposed to be because a free SH group was left on a carrier polymer immediately after the carrier polymer was treated with DTT and fractionated by the column. After the reaction was completed, a S-300HR Column was used to collect from the reaction solution a fraction including an antibody/carrier complex (A) (retention time of 24 to 44 minutes) as shown in Figure **7**.

### (Comparative Example 1)

In Comparative Example 1, a PBS solution having an anti-hCG antibody concentration of 10 mg/ml was brought into contact with DTSSP having the equivalents of 70, 100, and 200 to the antibody, as in Example 1. After the reaction was completed, the S-300HR Column was used to fractionate a fraction near the retention time of 45 minutes, so as to obtain antibody polymers (B), (C), and (D).

### (Measurement of subject substance with immunochromatography)

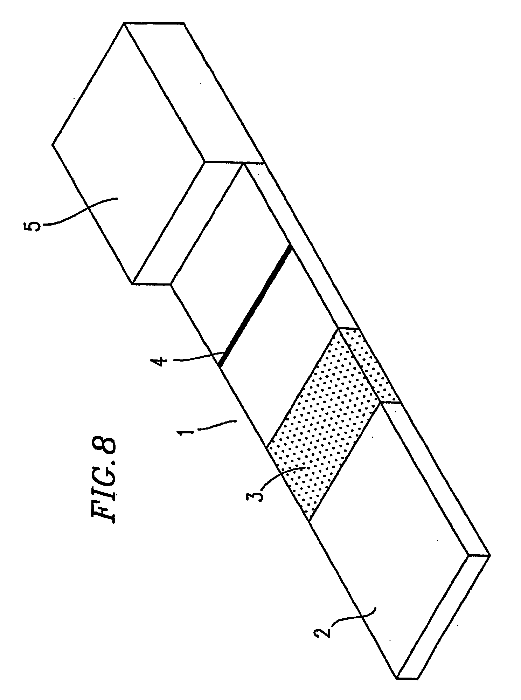

Immunochromatography, which is an immunological measurement method, was performed for a sample including hCG as a subject substance, using the antibody/carrier complex of Example 1 or the antibody polymer of Comparative Example 1, so as to measure hCG. This measurement of hCG is described with reference to Figure **8**. Figure **8** is a perspective view showing an immunochromatography device used for measurement.

An immunochromatography device including a porous carrier 1 shown in Figure **8** was used in the measurement. The antibody/carrier complex or antibody polymer was labeled with cyanine dye having a chemical structure shown below in Formula 1 (hereinafter, referred to as SLIC3), and carried in a labeling section **3** to a certain amount as a labeled antibody. 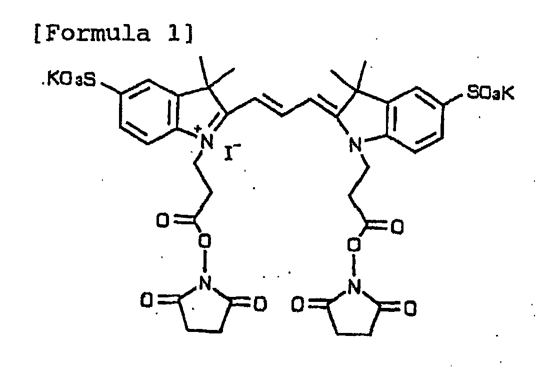 In a determination section **4**, an unpolymerized anti-hCG antibody was immobilized as an immobilized antibody. A PBS solution including hCG as an antigen was used as a sample. Such a sample having a hCG concentration of 0, 40, 400, 4,000, or 40,000 IU was added to a predetermined amount in a sample introduction section **2**. The sample moves toward a liquid absorbing section **5** via the labeling section **3** and determination section **4**. When the sample passes through the labeling section **3**, the labeled antibody carried in the labeling section **3** is dissolved into moisture of the sample, and begins to move together with the sample. Thereafter, the dissolved labeled antibody passes through the determination section **4**. If a specific bonding reaction of antigen and antibody is positive, the immobilized antibody and the labeled antibody are put into reaction via the antigen in the determination section **4**, so that the determination section **4** is stained. If negative, no reaction occurs, so that the labeled antibody passes through the determination section **4** and then is absorbed by the liquid absorbing section **5**. The intensity of color developed in the determination section **4** was measured using a reflection absorbance measurement device (CS9300PC produced by Shimadzu Corporation), where reflection absorption value for incident light at 550 nm (wavelength absorbed by SLIC3) was measured.

Table 1 below shows measurement results obtained for the antibody polymer of Comparative Example 1. Data value is a reflection absorption value. As the data value increases, the intensity of color increases. That is, the data value indicates the intensity of the affinity of the antibody, i.e., functions as an index which indicates the degree of affinity. The "ND" means that color of the dye was not detected, i.e., the affinity was not obtained. PBS was used as a negative control. The affinity of the unpolymerized antibody used was obtained only in the concentration range up to about 400 IU.

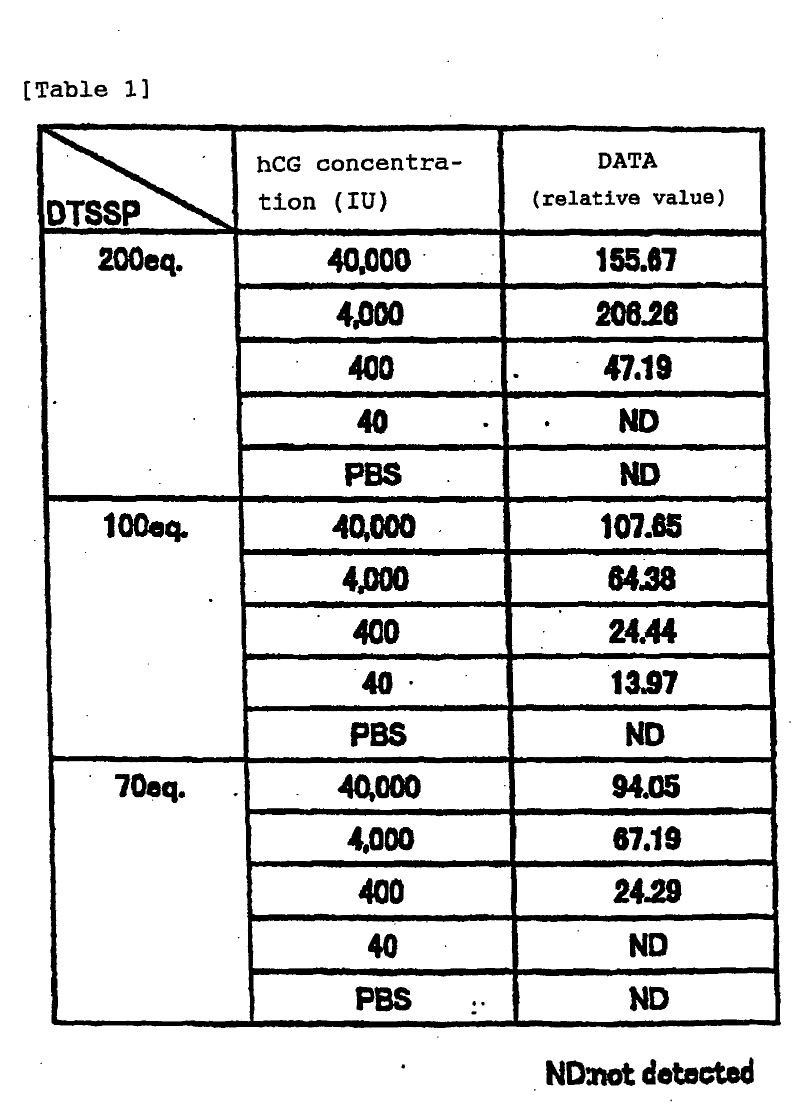

As shown in Table 1, in the antibody polymer (B) or (D) which was reacted using DTSSP having an equivalent of 70 or 200 with respect to the antibody, the affinity was only obtained to hCG having a concentration down to 400 IU. However, in the antibody polymer (C) which was put into reaction using DTSSP having the equivalent of 100 with respect to the antibody, affinity to hCG having a concentration down to 40 IU was obtained.

In the case where the equivalent of DTSSP is increased to the equivalent of 200 or more, the polymerization reaction further advances so that precipitate is generated. As a result, the affinity of the antibody is reduced to lower than that obtained before polymerization.

The same measurement was performed on the antibody/carrier complex (A) of Example 1. The affinity to a hCG having a concentration down to 40 IU was obtained. This affinity is substantially equal to that of the antibody polymer (C) produced using DTSSP which has the same equivalent. Considering that the total amount of protein carried in the labeling section 3 is substantially the same, it is understood that in the antibody/carrier complex (A) of Example 1, substantially the same affinity can be achieved with a smaller amount of antibody, in comparison to the antibody polymer (C) of the Comparative Example.

In the above process of producing the labeled antibody. labeling was performed after an antibody/carrier complex was produced. However, an antibody/carrier complex where only a carrier portion is labeled can be produced by labeling a carrier polymer and then bringing an antibody polymer into reaction with the labeled carrier polymer. As a result, the possibility that an antigen-antibody reaction is inhibited by a directly labeled antibody is reduced. Accordingly, the range of affinity to an antigen in an antigen-antibody reaction of the antibody/carrier complex can be extended towards higher affinity.

In the above, the degree of polymerization of antibody in an obtained antibody/carrier complex is controlled by controlling the equivalent of a polyfunctional reagent used in a reaction, whereby the reactivity of the antibody/carrier complex to an antigen is adjusted. However, the present invention is not limited to this, but various parameters can be used. For example, the reaction temperature, reaction time, and concentration of antibody in a reaction of an antibody with the polyfunctional reagent, etc., may be controlled.

Further, the Sephacryl S-300HR Column or Sephadex G-25 Column was used for purifying a polymerized antibody, but the present invention is not limited to these columns. Any other column may be used so long as the column can perform purification of macromolecules. For example, a column which is used for gel filtration in liquid chromatography, and which can separate an antibody typically having a molecular weight of 150,000 and a protein having a molecular weight several times larger than that of the antibody, may be used. Furthermore, a purification method utilizing electrophoresis may be used in substitution for HPLC. Still further, by modifying a reaction performed with a reagent and a reaction temperature, purification can be performed using a precipitation method, a re-crystallization method, etc.

### INDUSTRIAL APPLICABILITY

According to an antibody/carrier complex of the present invention, the reactivity of an antigen-antibody reaction can be changed without being limited to an inherent affinity of an antibody itself to an antigen while the amount of the antibody used is small. Further, according to a method for adjusting the reactivity of an antigen-antibody reaction of the present invention, the reactivity of the antigen-antibody reaction can readily be changed without producing a new antibody.

By using the method of the present invention, the measurement affinity in analysis, measurement, and assay utilizing an antigen-antibody reaction can be controlled so as to be a desired value. Thus, the method of the present invention is useful especially in the field of medicine.

## Claims

1. An antibody/carrier complex including at least one antibody, the antibody/carrier complex including a polymer structure wherein each antigen binding site of the antibody is allowed to be arranged so as to react with an antigen.

2. An antibody/carrier complex according to claim 1, wherein the polymer structure is an antibody polymer or carrier polymer.

3. An antibody/carrier complex according to claim 1, wherein the carrier is selected from a group consisting of protein, carbohydrate, and lipid.

4. An antibody/carrier complex according to claim 1, wherein the carrier is bonded to a Fc-site of the antibody.

5. A method for producing the antibody/carrier complex recited in claim 1, comprising a step of copolymerizing an antibody and a carrier using a polymerization reagent.

6. A method for producing the antibody/carrier complex recited in claim 1, comprising: a step of polymerizing a carrier using a polymerization reagent so as to form a carrier polymer; and a step of bonding an antibody to the carrier polymer.

7. A method for producing the antibody/carrier complex recited in claim 1, comprising: a step of polymerizing an antibody using a polymerization reagent so as to form an antibody polymer; and a step of bonding a carrier to the antibody polymer.

8. A method for producing the antibody/carrier complex recited in claim 1, comprising: a step of polymerizing a carrier using a polymerization reagent so as to form a carrier polymer; a step of polymerizing an antibody using a polymerization reagent so as to form an antibody polymer; and a step of bonding the antibody polymer and the carrier polymer.

9. A method for producing the antibody/carrier complex according to any of claims 5-8, wherein the polymerization reagent is 3,3'-dithiobis(sulfosuccinimidylpropionate) or dithiothreitol.

10. A method for adjusting the reactivity of an antigen-antibody reaction, comprising steps of:
in a reaction of an antibody and a polymerization reagent, changing a parameter selected from a group consisting of the equivalent of the polymerization reagent with respect to the antibody, reaction temperature, reaction time, and the concentration of antibody, so as to obtain a plurality of antibody polymers having different degrees of polymerization;
bonding the plurality of antibody polymers to carriers so as to obtain a set of antibody/carrier complexes; and
selecting an antibody/carrier complex which reacts with an antigen at a desired degree of reaction, from the set of antibody/carrier complexes.

11. An immunological measurement method, comprising a step of bringing a subject substance in a sample and the antibody/carrier complex recited in one of claims 1-4 into an antigen-antibody reaction.
